# NEUE EUROPÄISCHE PATENTSCHRIFT

(11) **EP 2 136 129 B2**
(45) Veröffentlichungstag und Bekanntmachung des Hinweises auf die Entscheidung über den Einspruch: **18.12.2019**
(45) Hinweis auf die Patenterteilung: 19.01.2011
(21) Anmeldenummer: 08014768.9
(22) Anmeldetag: 20.08.2008
(51) Int. Cl.: F21S 8/00, F21V 23/00

(54) **Operationsleuchte mit abstandsabhängiger Helligkeitsregelung**
Operating light with distance-dependant brightness control
Eclairage d'opération doté d'un réglage de la luminosité dépendant de l'intervalle

(30) Priorität: 20.06.2008 EP 08011296
(43) Veröffentlichungstag der Anmeldung: 23.12.2009
(73) Patentinhaber: TRUMPF Medizin Systeme GmbH + Co. KG, 07318 Saalfeld (DE)
(72) Erfinder: Rosenheimer, Rouven, 85614 Kirchseeon (DE); Marka, Rudolf, 85737 Ismaning (DE)
(74) Vertreter: Prüfer & Partner mbB Patentanwälte · Rechtsanwälte

(56) Entgegenhaltungen:
- EP-A- 0 299 196
- EP-A- 0 422 331
- WO-A2-03/083359
- DE-A1- 19 839 827
- JP-A- 2004 288 474
- US-A- 5 383 105

## Beschreibung

Die Erfindung betrifft eine Operationsleuchte, die ein Leuchtfeld auf dem Operationsfeld erzeugt, und eine Einrichtung zur Einstellung der Leuchtintensität der Leuchtmittel in Abhängigkeit von dem Abstand zwischen der Operationsleuchte und dem Operationsfeld.

Eine Möglichkeit der Ausführung von Operationsleuchten sind so genannte Großspiegelleuchten. Hierbei ist die Lichtquelle eine Halogenlampe oder Gasentladungslampe, die im Brennpunkt eines großen Reflektors mit einem Durchmesser von ca. 500 mm bis 1000 mm angeordnet ist. Durch eine Verschiebung der Lichtquelle auf der Mittelachse des Reflektors, und damit mehr oder weniger aus dem oder in den optimalen Brennpunkt des Reflektors, wird der Durchmesser des Leuchtfelds, d.h. der beleuchtete Durchmesser im Operationsfeld vergrößert oder verkleinert, und der Fokuspunkt verändert, d.h. der Abstand der hellsten beleuchteten Stelle, in der sich die reflektierten Lichtstrahlen schneiden, von dem Leuchtenkörper der Operationsleuchte auf einer Mittelachse des Leuchtenkörpers verändert. Hierfür ist eine aufwändige Mechanik erforderlich, die eine prozesssichere leichtgängige Bedienung ohne großen Betätigungsaufwand des Bedieners, ermöglicht. Dadurch ändert sich die Helligkeit, da die Lichtmenge auf eine größere Fläche verteilt wird und die Verteilung der Leuchtstärke über dem Durchmesser des Leuchtfelds, und somit auch die Leuchtstärke im Zentrum des Leuchtfelds.

Eine andere Ausführung sind aufgelöste Lichtsysteme. Hierbei umfasst die Operationsleuchte in der Regel einen zentralen Scheinwerfer oder ein zentrales Lichtmodul, der/das starr am Leuchtenkörper befestigt ist und mehrere Scheinwerfer oder Lichtmodule in einer ringförmigen Anordnung um den zentralen Scheinwerfer oder das zentrale Lichtmodul aufweist. Die Veränderung der Richtung des Lichtaustritts aus den äußeren Scheinwerfern oder Lichtmodulen ist über radial verschwenkbare Leuchtmittel oder Reflektoren realisiert, oder die gesamten Scheinwerfer oder Lichtmodule sind radial schwenkbar verstellbar, so dass der Fokuspunkt der Lichtstrahlen aus den äußeren Scheinwerfern oder Lichtmodulen seinen Abstand vom Leuchtenköper auf der Mittelachse verändert. Auch dadurch ändert sich die gesamte Helligkeit, und die Verteilung der Leuchtstärke über dem Durchmesser des Leuchtfelds, und somit auch die Leuchtstärke im Zentrum des Leuchtfelds. Dabei ist ebenfalls eine aufwändige Mechanik zur synchronen, prozesssicheren Verstellung der äußeren Leuchtmittel, Reflektoren oder Lichtmodulen erforderlich.

Die Offenlegung EP-A-0299196 offenbart z.B. eine Operationsleuchte, die mit Hilfe eines Ultraschallmesssystems, das den Abstand der Operationsleuchte zur Operationsstelle misst und die Leuchtmittel entsprechend verstellt, so dass die Grundeinstellung des Beleuchtungsbereichs, insbesondere die Leuchtfeldbreite möglichst unverändert bleibt. Eine Anpassung der Leuchtstärke, die dazu dient, die Helligkeit konstant zu halten, ist nicht offenbart.

Weiterhin zeigt die EP-A-0422331 eine Operationsleuchte, bei der nicht nur wie oben angegeben der Abstand der Operationsleuchte zur Operationsstelle gemessen wird, sondern über einen Auslösemechanismus veranlasst wird, dass die Winkelstellungen sämtlicher Gelenke des Aufhängesystems über Winkelgeber gespeichert werden, sowie der Abstand zwischen dem Leuchtengehäuse und dem eingestellten Beleuchtungsbereich bestimmt und abgespeichert wird. Dadurch kann die genaue Lage des Beleuchtungsbereichs der Operationsstelle im Raum erfasst werden. Bei einer räumlichen Verlagerung der Leuchte wird durch erneutes Erfassen der Winkelstellung sämtlicher Winkel die neue Position des Leuchtengehäuses erfasst und mit dem abgespeicherten Sollwert verglichen. Die drei dem Leuchtengehäuse nächstliegenden Gelenke werden dann über Stellmotoren so eingestellt, dass die Leuchte wieder auf die abgespeicherte Operationsstelle gerichtet ist und die Fokussierung der Operationsleuchte wird auf den nun errechneten Abstand zwischen Leuchtengehäuse und Operationsstelle eingestellt.

Eine weitere Art von Operationsleuchten ist ohne die Verstellmöglichkeit des Leuchtfelddurchmessers und des Abstands des Fokuspunkts ausgeführt. Hierbei sind die lichttechnischen Daten, Leuchtfelddurchmesser und Fokuspunkt für einen Arbeitspunkt optimal eingestellt. Eine Veränderung des Leuchtfelddurchmessers ist hier nur durch eine Veränderung des Abstands des Leuchtenkörpers vom Operationsfeld möglich.

Bei diesen Ausführungen von Operationsleuchten ändert sich die Beleuchtungsstärke im Zentrum des Leuchtfelds bei einer Veränderung des Abstands zwischen der Operationsleuchte und dem Operationsfeld zum einen, da sich der Abstand von der Strahlungsquelle verändert, was zu einer Abnahme der Strahlungsintensität bei einer Vergrößerung der Entfernung oder Zunahme der Strahlungsintensität bei einer Verringerung der Entfernung führt, und zum anderen, da die Verteilung der Leuchtstärke im Leuchtfeld nicht mehr normgerecht ist, und somit die Leuchtstärke im Zentrum abnimmt.

Das Gebrauchsmuster DE-A-20316756 offenbart eine Operationsleuchte mit einem Helligkeitssensor (Fotodiode), der sich am Rand des Reflektors befindet, oder einer CCD-Kamera, die sich an der Operationsleuchte oder an einer anderen Stelle befindet. Mit Hilfe des Helligkeitssensors wird das Licht, das von der Operationsstelle reflektiert wird, erfasst, und bei einer Veränderung des reflektierten Lichts wird die auszustrahlende Beleuchtungsstärke errechnet und an die Beleuchtungsquelle weitergegeben. Alternativ wird mit Hilfe der Kamera die derzeit herrschende Helligkeit erfasst und an die Steuerung der Kamera weitergegeben. Nach einer Positionsänderung der Operationsleuchte können sich jedoch die Reflexionseigenschaften des Gewebes bei der Verwendung des Helligkeitssensors und der für die Helligkeitsauswertung zu betrachtende Bildausschnitt jedoch stark verändern, so dass eine Korrektur der Helligkeit nicht mehr zuverlässig möglich ist.

Es ist Aufgabe der Erfindung, eine Operationsleuchte zur Verfügung zu stellen, die kostengünstig die Möglichkeit bietet, die Beleuchtungsstärke bei einer Veränderung des Abstands zwischen der Operationsleuchte und dem Operationsfeld selbsttätig konstant zu halten.

Die Aufgabe wird mit den Merkmalen des Anspruchs 1 gelöst. Weiterbildungen der Erfindung sind in den Unteransprüchen beschrieben.

Die Operationsleuchte bietet durch das Aufweisen einer Sensoreinrichtung zum Messen des Abstands zwischen der Operationsleuchte und dem Operationsfeld und einer Ansteuerung der Leuchtmittel die Möglichkeit, die Leuchtintensität der Leuchtmittel so zu verändern, dass die Helligkeit im Operationsfeld bei einer Veränderung des Abstands konstant bleibt. Dies erfolgt ohne mechanische Verstellmittel.

Die Erfindung wird anhand eines Ausführungsbeispiels unter Bezugnahme auf die beigefügten Zeichnungen erläutert.
- Fig. 1: ist eine perspektivische Ansicht eines Ausführungsbei- spiels der erfindungsgemäßen Operationsleuchte.
- Fig. 2: ist eine Schnittansicht eines Leuchtenkörpers mit der Anordnung einer Sensoreinrichtung zur Abstandserfas- sung.

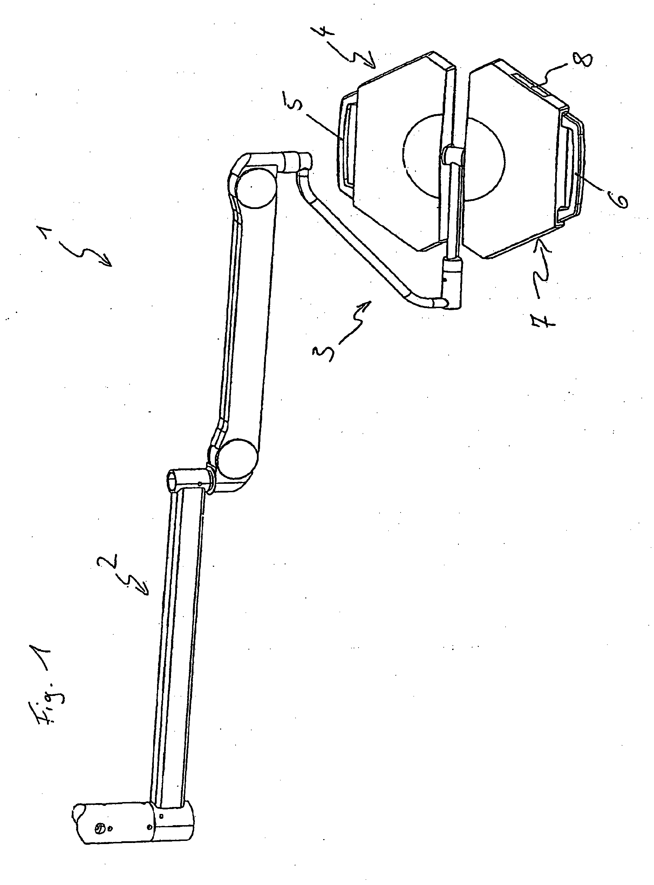

Fig. 1 zeigt eine perspektivische Ansicht eines Ausführungsbeispiels der erfindungsgemäßen Operationsleuchte 1. Die Operationsleuchte 1 umfasst ein Tragsystem 2, eine Aufhängevorrichtung 3 und einen Leuchtenkörper 4. Das Tragsystem 2 wird an einer Raumdecke, einer Wand oder einem fahrbaren Ständer befestigt. Durch das Tragsystem 2 und die Aufhängevorrichtung 3 ist der Leuchtenkörper 4 innerhalb des Aktionsradius in jeder beliebigen räumlichen Stellung und Orientierung positionierbar. Auf der nicht gezeigten, gegenüberliegenden Seite des Leuchtenkörpers 4 ist auf nahezu der gesamten Fläche eine Lichtaustrittsöffnung 15 angeordnet, die im Betrieb auf ein Operationsfeld, das sich in einem bestimmten Abstand befindet, gerichtet ist.

Zum unsterilen Positionieren des Leuchtkörpers 4 sind an den beiden Hälften des Leuchtenkörpers 4 je ein Griff 5, 6 angebracht. Die beiden Hälften des Leuchtenkörpers 4 sind drehfest miteinander verbunden, so dass sich beim Verschwenken der einen Hälfte die andere Hälfte mitbewegt, um die Lichtaustrittsflächen immer in einer Ebene zu halten.

Zum sterilen Positionieren des Leuchtkörpers ist ein Handgriff im Zentrum der Lichtaustrittsöffnung angeordnet. Über den Handgriff wird eine Hülse gesteckt, die sterilisierbar ist.

Innerhalb des Leuchtenkörpers 4 ist eine Steuerungsvorrichtung 7 angeordnet. Die Steuerungsvorrichtung 7 muss nicht zwingend in dem Leuchtenkörper 4 angeordnet sein, sondern kann auch in einem separaten Gehäuse, das am Leuchtenkörper 4 oder an der Aufhängevorrichtung 3 angeordnet ist, untergebracht sein. Alternativ besteht auch die Möglichkeit, dass sich die Steuerungsvorrichtung 7 in einer nicht gezeigten externen Bedieneinheit befindet, die sich in einer Medizinischen Versorgungseinheit oder in/an einer Wand befindet.

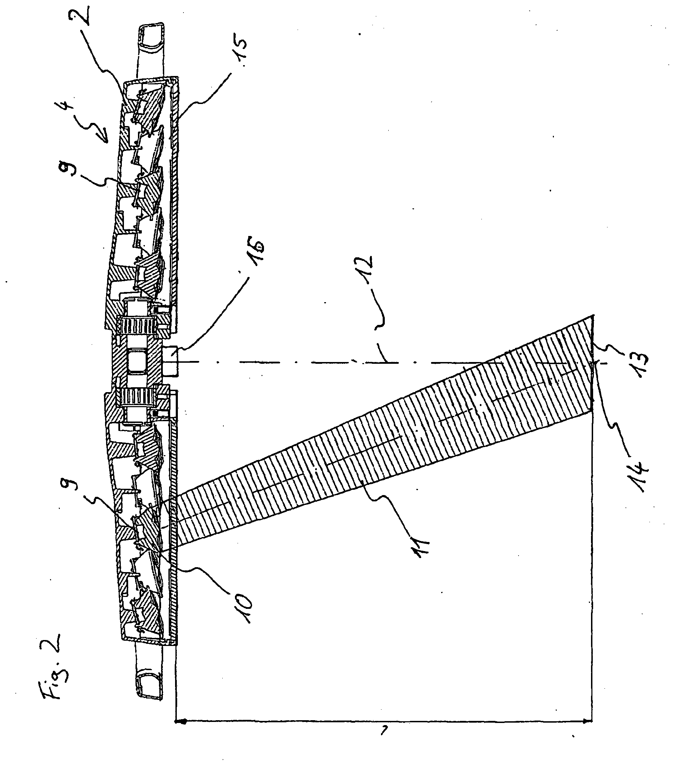

An der Außenseite des Leuchtenkörpers 4 ist eine Bedienvorrichtung 8 angeordnet. Die Bedienvorrichtung 8 kann sich aber auch in einem separaten Gehäuse befinden, das sich beispielsweise am Leuchtenkörper 4, an der Aufhängevorrichtung 3 oder in einer Medizinischen Versorgungseinheit oder in/an einer Wand befindet. Fig. 2 ist eine Schnittansicht eines Leuchtenkörpers 4, in dem eine Mehrzahl von Leuchtmitteln 9 vorgesehen ist. Der Leuchtenkörper 4 hat eine Mittelachse 12, die senkrecht auf einer Ebene steht, in der die Leuchtmittel 9 angeordnet sind. Die Leuchtmittel 9 sind ringförmig oder entsprechend der Form des Leuchtenkörpers 4 angeordnet.

In einer alternativen Ausführungsform ist auch das Vorsehen von nur einem Leuchtmittel möglich.

Jedes Leuchtmittel 9 ist mit einer Vorrichtung zum Bündeln des Lichts, hier einem Refraktor 10, ausgestattet. Anstelle der separaten Refraktoren 10 können auch separate Reflektoren oder Leuchtmittel mit integrierten Mitteln zum Bündeln des Lichts verwendet werden.

Das gebündelte Licht der Leuchtmittel 9, tritt in einem Lichtstrahl 11 aus dem Refraktor 10 aus. Ein Lichtstrahl 11 beleuchtet jeweils ein gesamtes Leuchtfeld 13.

In einer alternativen Ausführungsform bildet die Operationsleuchte mehrere Leuchtfelder 13 in verschiedenen Abständen, d.h. der Schnittpunkt der Lichtstrahlbündel 11 liegt in Ebenen in verschiedenen Abständen. In einer speziellen Ausführungsform betragen die Abstände der Ebenen, bzw. der Leuchtfelder 13 vom Leuchtenkörper 4, 90 cm, 100 cm und 110 cm. Die Leuchtmittel 9, die die verschiedenen Leuchtfelder 13 bilden, sind steuerungstechnisch gruppenweise zusammengefasst und werden in Abhängigkeit von dem Abstand des Leuchtenkörpers 4 von dem Operationsfeld aktiviert.

Die Leuchtmittel 9 mit den Refraktoren 10 sind geneigt angeordnet, so dass die Achsen der Lichtstrahlbündel 11 die Mittelachse 12 jeweils in jeweils einem Schnittpunkt 14, der in der Ebene des zugehörigen Leuchtfelds 13 liegt, schneiden.

Die Leuchtmittel 9 sind in der Ebene gleichmäßig verteilt, um ein Hindernis, das zwischen den Leuchtenkörper 4 und das Operationsfeld eingebracht wird und dadurch ein Lichtstrahlenbündel unterbricht, was eine Schattenbildung zur Folge hat, zu unterleuchten und somit die Schattenbildung zu verhindern oder abzuschwächen.

In dem Leuchtenkörper 4 ist die nicht gezeigte Steuerungsvorrichtung 7 angeordnet. Die Steuerung weist Einrichtungen zum Dimmen und Ein- und Ausschalten der Leuchtmittel 9, wie z.B. Stromregler, Mittel zum Übertragen von Schalt- und Einstellinformationen der Schalt- und Einstellelemente der Bedienvorrichtung 8, einen Speicherbereich zum Abspeichern von Betriebsparametern und eine CPU, die aus den Schalt- und Einstellinformationen, an Hand der abgespeicherten Betriebsparameter, die erforderlichen Einstellungen für die Mittel zum Dimmen und Ein- und Ausschalten der Leuchtmittel berechnet oder bestimmt, auf.

Im zentralen Bereich des Leuchtenkörpers 4 ist auf der Seite der Lichtaustrittsfläche 15 eine Sensoreinrichtung 16 angeordnet. Diese Sensoreinrichtung 16 erfasst den Abstand zwischen der Lichtaustrittsfläche 15 des Leuchtenkörpers 4 und dem Operationsfeld, wandelt diese Information in elektrische Signale um und gibt diese an die Steuerungsvorrichtung 7 weiter. Die Sensoreinrichtung 16 verwendet zur Abstandsmessung einen Laser-Abstandsmesser, der eine punktgenaue Abstandsmessung ermöglicht, die nicht von der Farbe oder der Struktur der Oberfläche des Operationsfelds abhängig ist.

Der Laser-Abstandsmesser sendet während des Messvorgangs einen Laserstrahl mit einem roten oder in einer anderen Ausführungsform grünen Licht, das besser erkennbar ist, in das Zentrum des Operationsfelds. Da der Laserstrahl eine Wellenlänge im sichtbaren Bereich aufweist, kann der Laserstrahl auch zur visuellen Kontrolle der Orientierung der Operationsleuchte 1 verwendet werden, d.h. ob das Zentrum des Leuchtfelds 13 im Zentrum des Operationsfeld liegt. Da das Licht, das durch die Operationsleuchte 1 zur Beleuchtung des Operationsfelds ausgestrahlt wird, jedoch ebenfalls einen Anteil bei dieser Wellenlänge aufweist, kann die Abstandsmessung gestört werden. Aus diesem Grund wird während der Messung das Licht, das zur Beleuchtung des Operationsfelds dient, abgedimmt.

Um das Dimmen zu vermeiden, gibt es in einer weiteren Ausführungsform eine Laserstrahlquelle, die Licht in einem Wellenlängenbereich abstrahlt, der nicht im sichtbaren Bereich, sondern im Infrarot (IR)- oder Ultraviolett (UV)-Bereich liegt. Operationsleuchten 1 strahlen auf Grund der Verwendung von Filtern, oder von Leuchtmitteln, die nur einen sehr geringen Anteil von ein Licht in diesem Wellenlängenbereich abstrahlen, wie z.B. LEDs, in diesem Wellenlängenbereich kein störendes Licht aus.

Zur besseren Ausrichtung der Operationsleuchte 1 bei einer ungestörten Abstandsmessung gibt es in einer zusätzlichen Ausführungsform eine Kombination einer Laserstrahlquelle, die sichtbares Licht zum Ausrichten ausstrahlt, und einer Laserstrahlquelle, die Licht im UV- oder IR-Bereich zur Abstandsmessung ausstrahlt.

In alternativen Ausführungsformen kann die Sensoreinrichtung 16, oder Teile der Sensoreinrichtung 16 auch in anderen Bereichen des Leuchtenkörpers oder am Tragsystem 2 angeordnet sein. Innerhalb des Leuchtenkörpers 2 ist ein (nicht gezeigter) Auslösemechanismus, hier ein Beschleunigungssensor, vorgesehen, der eine Bewegung des Leuchtenkörpers erfasst und nach Abschluss der Bewegung ein Signal zur Korrektur, also zur Neuberechnung der Leistungswerte und die Übertragung der Leistungswerte an die Leuchtmittel 9 gibt.

Das Auslösen der Korrektur kann auch mit Hilfe anderer Auslösemechanismen, z.B. einem Schalter, der erfasst, ob ein Griff (steril oder unsteril) der Operationsleuchte berührt wurde, oder einem Bedienelement an der Bedienvorrichtung 8, ausgeführt werden.

Die Steuerungsvorrichtung 7 ist mit den Leuchtmitteln 9 verbunden, die gruppenweise angesteuert werden. Eine Gruppe wird aus mehreren Leuchtmitteln 9 gebildet, die mit den gleichen Leistungsparametern angesteuert werden. Es sind mehrere Gruppen möglich, deren Lichtstrahlen 11 der Leuchtmittel 9 auf jeweils den gleichen Schnittpunkt 14 gerichtet sind.

Weiterhin ist die Steuerungsvorrichtung 7 mit der Sensoreinrichtung 16 verbunden.

Die Steuerungsvorrichtung 7 ist ebenfalls mit der Bedienvorrichtung 8 verbunden. An der Bedienvorrichtung 8 ist
- ein Element zum Ein-/Ausschalten,
- ein Element zur Abstandseinstellung (Abspeichern des momentanen Abstands) und
- ein Element zur Helligkeitseinstellung vorgesehen.

Das Element zum Ein-/Ausschalten schaltet die Operationsleuchte 1 von einem Standby-Modus, in der die Leuchtmittel 9 nicht leuchten, in einen Betriebsmodus. Dabei werden die Leuchtmittel 9 entsprechend der Einstellung der Einstellelemente betrieben. Zum vollständigen Ausschalten durch Abschalten der Stromversorgung ist ein nicht gezeigter externer Hauptschalter vorgesehen.

Das Element zur Abstandseinstellung gibt an die Steuerungsvorrichtung 7 die Information, das durch die Sensoreinrichtung 16 abgegebene Signal durch die Steuerungsvorrichtung 7 zu verarbeiten.

Im Betrieb wird der Leuchtenkörper 2 der Operationsleuchte 1 auf das Operationsfeld eingestellt, so dass optimale Beleuchtungsbedingungen (Helligkeit, Beleuchtungswinkel und Fokussierung) vorliegen. Anschließend wird das Element zur Abstandseinstellung an der Bedienvorrichtung 8 betätigt und damit veranlasst, dass der Abstand des Leuchtenkörpers 2 von dem Operationsfeld erfasst wird. Bei einer Veränderung der Position des Leuchtenkörpers 2 auf Grund eines Erfordernisses, dass die Beleuchtungsrichtung verändert werden muss, erkennt der Beschleunigungssensor, dass der Leuchtenkörper 2 bewegt wird und überträgt ein entsprechendes Signal an die Steuerungsvorrichtung 7. Nach Beendigung der Bewegung wertet die Steuerungsvorrichtung 7 die durch die Sensoreinrichtung 16 übermittelten Signale bezüglich des nun vorliegenden Abstands aus, wählt zugehörigen Korrekturwerte aus, die in dem Speicherbereich der Steuerungsvorrichtung 7 abgelegt sind, und ermittelt somit die erforderlichen Leistungswerte für die Leuchtmittel 9, die an die Einrichtungen zum Dimmen und Ein-und Ausschalten der Leuchtmittel 9 weitergeben werden. Die Beleuchtungsstärke wird damit so gewählt, dass die Helligkeit im Operationsfeld der Helligkeit vor der Positionsänderung des Leuchtenkörpers entspricht.

Die Korrekturwerte für die verschiedenen Abstände werden für einzelne Betriebsbedingungen (Abstand und Beleuchtungssituation) empirisch ermittelt und als Kennfeld in dem Speicherbereich der Steuerungsvorrichtung 7 abgespeichert.

Bei Bedarf kann die Operationsleuchte nachträglich kalibriert werden. Hierzu ist die Steuerungsvorrichtung 7 mit nachträglich veränderbaren Speicherbausteinen versehen und die Software so gestaltet, dass durch die Abfolge einer definierten Sequenz die Helligkeit bei verschiedenen Abständen eingestellt wird und dann durch Ausführen einer Tastenkombination die Korrekturwerte abgespeichert werden.

In einer alternativen Ausführungsform kann die Sensoreinrichtung 16 auch einen Ultraschallsensor zur Erfassung des Abstands verwenden. Hierbei muss allerdings in Kauf genommen werden, dass der Ultraschallsensor einen Schallkegel bildet, der in einem in dieser Anwendung auftretenden Abstand einen Durchmesser von 30 cm bis 40 cm ausbildet, und somit die Abstandserkennung ungenauer durchgeführt wird, als bei der Verwendung eines Laser-Abstandsmessers.

Eine weitere Alternative im Hinblick auf den Sensor ist ein Infrarotsensor, der zwar kostengünstig, aber das Erfassungsergebnis des Abstands abhängig von der Farbe der erfassten Fläche ist. Bei Operationen treten je nach Verlauf jedoch die verschiednen Bedingungen auf, dass sich entweder grüne oder blaue Abdecktücher, Haut (benetzt mit orange-farbigem Desinfektionsmittel), rotes Muskel- oder weißes Fettgewebe oder Knochen im Leuchtfeld 13 befinden. Daher ist die Erfassung ungenauer.

In weiteren alternativen Ausführungsformen wird nicht die Leistung der Leuchtmittel 9 verändert, um die Leuchtintensität des abgestrahlten Lichts zu verändern, sondern die Lichtstrahlen 11 durch mechanische Blenden oder dimmbare Folien so abgeschwächt, dass die für die Konstanthaltung der Helligkeit erforderliche Lichtstärke erreicht wird.

## Patentansprüche

1. Operationsleuchte (1), umfassend
einen Leuchtenkörper (4)
mit einer Lichtaustrittsfläche (15), und
mit mindestens einem Leuchtmittel (9), geeignet zum Ausstrahlen eines Lichtstrahls (11) auf ein Operationsfeld (13),
eine Sensoreinrichtung (16) zum Erfassen eines Abstands zwischen dem Leuchtenkörper (4) und dem Operationsfeld,
eine Steuerungsvorrichtung (7), und
eine Einrichtung zum Dimmen und Ein- und Ausschalten der Leuchtmittel (9),
**dadurch gekennzeichnet, dass** die Steuerungsvorrichtung (7) dazu angepasst ist, den von der Sensoreinrichtung (16) erfassten Abstand zwischen der Lichtaustrittsfläche (15) des Leuchtenkörpers (4) und dem Operationsfeld auszuwerten und die für den erfassten Abstand erforderlichen Leistungswerte für die Leuchtmittel (9) an die Einrichtung zum Dimmen und Ein- und Ausschalten der Leuchtmittel (9) weiterzugeben, so dass eine Leuchtintensität der Leuchtmittel (9) so verändert wird, dass eine Helligkeit in dem Operationsfeld bei einer Veränderung des Abstands konstant bleibt.

2. Operationsleuchte gemäß Anspruch 1, dadurch gekennzeicnnet, dass eine zentrale Steuerungsvorrichtung (7) vorhanden ist, die mit der Sensoreinrichtung (16) zum Erfassen des Abstands, und den Vorrichtungen zur Steuerung der Intensität des ausgestrahlten Lichts verbunden ist.

3. Operationsleuchte gemäß einem der vorangehenden Ansprüche, **dadurch gekennzeichnet, dass** die Einrichtung zur Steuerung der Intensität des ausgestrahlten Lichts ein elektrischer Leistungsregler zum Ansteuern der Lichtquelle (9) ist.

4. Operationsleuchte gemäß einem der Ansprüche 2 oder 3, **dadurch gekennzeichnet, dass** die zentrale Steuerungsvorrichtung (7) Vorrichtungen zum Abspeichern von Daten oder von Algorithmen aufweist.

5. Operationsleuchte gemäß einem der vorangehenden Ansprüche, **dadurch gekennzeichnet, dass** ein Auslösemechanismus vorhanden ist, der eine Veränderung der Intensität des ausgestrahlten Lichts auslöst.

6. Operationsleuchte gemäß Anspruch 5, **dadurch gekennzeichnet, dass** der Auslösemechanismus einen Beschleunigungssensor enthält.

7. Operationsleuchte gemäß Anspruch 5, **dadurch gekennzeichnet, dass** der Auslösemechanismus einen Schalter in einem Handgriff enthält.

8. Operationsleuchte, gemäß einem der vorangehenden Ansprüche, **dadurch gekennzeichnet, dass** wenigstens eine Einrichtung zum Kalibrieren der Steuerung vorhanden ist.

9. Operationsleuchte gemäß einem der Ansprüche 1 bis 8, **dadurch gekennzeichnet, dass** die Sensoreinrichtung zum Messen des Abstands ein Ultraschallsensor ist.

10. Operationsleuchte gemäß einem der Ansprüche 1 bis 8, **dadurch gekennzeichnet, dass** die Sensoreinrichtung (16) zum Messen des Abstands ein Infrarotsensor ist.

11. Operationsleuchte gemäß einem der Ansprüche 1 bis 8, **dadurch gekennzeichnet, dass** die Sensoreinrichtung (16) zum Messen des Abstands ein Lasersensor ist.

12. Operationsleuchte gemäß Anspruch 11, **dadurch gekennzeichnet, dass** der Lasersensor Licht im sichtbaren Wellenlängenbereich ausstrahlt.

13. Operationsleuchte gemäß Anspruch 11, **dadurch gekennzeichnet, dass** der Lasersensor Licht im nicht sichtbaren Wellenlängenbereich ausstrahlt.

14. Operationsleuchte gemäß Anspruch 13, **dadurch gekennzeichnet, dass** die Operationsleuchte eine Laserstrahlquelle, die Licht im sichtbaren Bereich ausstrahlt, aufweist.

15. Operationsleuchte gemäß Anspruch 1, **dadurch gekennzeichnet, dass** mehrere Leuchtmittel (9) zu Gruppen zusammengefasst sind, deren Lichtstrahlbündel (11) jeweils einen Schnittpunkt in der Ebene des zugehörigen Leuchtfelds (13) aufweisen und die Leuchtfelder (13) verschiedene Abstände zum Leuchtenkörper (4) aufweisen.

## Claims

1. Surgical light (1) comprising
a lamp body (4)
having a light emergence face (15) and
at least one illuminant (9) suitable for radiating a light beam (11) to a surgical site (13),
a sensor device (16) for detecting a distance between the lamp body (4) and the surgical site,
a control device (7), and
a device for dimming and switching on and switching off of the illuminants (9), **characterized in that**
the control device (7) is adapted to analyze the distance, detected by the sensor device (16), between the light emergence face (15) of the light head (4) and the surgical site and to transmit the power output values for the illuminants (9) necessary for the detected distance to the device for dimming and switching on and switching off of the illuminants (9) so that an illumination intensity of the illuminants (9) is changed such that a brightness in the surgical site remains constant upon a change of the distance.

2. Surgical light according to claim 1, **characterized in that** a central control device (7) is provided which is connected to the sensor device (16) for detecting the distance and the devices for controlling the intensity of the radiated light.

3. Surgical light according to any of the preceding claims, **characterized in that** the device for controlling the intensity of the radiated light is an electrical output regulator for driving the illuminant (9).

4. Surgical light according to any of claim 2 or 3, **characterized in that** the central control device (7) comprises devices for storing data or algorithms.

5. Surgical light according to any of the preceding claims, **characterized in that** an actuator is provided which actuates a change of the intensity of the radiated light.

6. Surgical light according to claim 5, **characterized in that** the actuator comprises an acceleration sensor.

7. Surgical light according to claim 5, **characterized in that** the actuator comprises a switch in a handle.

8. Surgical light according to any of the preceding claims, **characterized in that** at least one device for calibrating the control is provided.

9. Surgical light according to any of claims 1 to 8, **characterized in that** the sensor device for measuring the distance is an ultrasonic sensor.

10. Surgical light according to any of claims 1 to 8, **characterized in that** the sensor device (16) for measuring the distance is an infrared sensor.

11. Surgical light according to any of claims 1 to 8, **characterized in that** the sensor device (16) for measuring the distance is a laser sensor.

12. Surgical light according to claim 11, **characterized in that** the laser sensor radiates light in the visible wavelength range.

13. Surgical light according to claim 11, **characterized in that** the laser sensor radiates light in the non-visible wavelength range.

14. Surgical light according to claim 13, **characterized in that** the surgical light comprises a laser beam source which radiates light in the visible wavelength range.

15. Surgical light according to claim 1, **characterized in that** multiple illuminants (9) are collected to groups, the pencils (11) of light beams of which having an intersection point in the plane of the related light field (13), respectively, and that the light fields (13) have different distances to the lamp body (4).

## Revendications

1. Luminaire d'opération (1), comprenant :
un corps de luminaire (4),
avec une surface de sortie de lumière (15), et
avec au moins un moyen d'éclairage (9), convenant pour irradier un rayon lumineux (11) sur un champ d'opération (13),
un dispositif capteur (16) pour détecter un espacement entre le corps de luminaire (4) et le champ d'opération,
un dispositif de commande (7), et
un dispositif pour graduer et mettre en service et hors service les moyens d'éclairage (9),
**caractérisé en ce que** le dispositif de commande (7) est adapté pour évaluer l'espacement, appréhendé par le dispositif capteur (16), entre la surface de sortie de lumière (15) du corps de luminaire (4) et le champ d'opération, et pour retransmettre au dispositif les valeurs de puissance nécessaires pour l'espacement appréhendé pour les moyens d'éclairage (9), pour graduer et mettre en service et hors service les moyens d'éclairage (9), de sorte qu'une intensité d'éclairage des moyens d'éclairage (9) est modifiée de sorte qu'une luminosité dans le champ d'opération reste constante lors d'une modification de l'espacement.

2. Luminaire d'opération selon la revendication 1, **caractérisé en ce qu'**est prévu un dispositif de commande (7) central, qui est relié au dispositif capteur (16) pour appréhender l'espacement, et aux dispositifs pour la commande de l'intensité de la lumière irradiée.

3. Luminaire d'opération selon l'une des revendications précédentes, **caractérisé en ce que** le dispositif pour la commande de l'intensité de la lumière irradiée est un régulateur de puissance électrique, pour commander la source lumineuse (9).

4. Luminaire d'opération selon l'une des revendications 2 ou 3, **caractérisé en ce que** le dispositif de commande (7) central présente des dispositifs pour mémoriser des données ou des algorithmes.

5. Luminaire d'opération selon l'une des revendications précédentes, **caractérisé en ce qu'**est prévu un mécanisme de déclenchement, qui déclenche une modification de l'intensité de la lumière irradiée.

6. Luminaire d'opération selon la revendication 5, **caractérisé en ce que** le mécanisme de déclenchement contient un capteur d'accélération.

7. Luminaire d'opération selon la revendication 5, **caractérisé en ce que** le mécanisme de déclenchement contient un interrupteur dans une poignée.

8. Luminaire d'opération selon l'une des revendications précédentes, **caractérisé en ce qu'**est prévu au moins un dispositif pour l'étalonnage de la commande.

9. Luminaire d'opération selon l'une des revendications 1 à 8, **caractérisé en ce que** le dispositif capteur pour mesurer l'espacement est un capteur à ultra-sons.

10. Luminaire d'opération selon l'une des revendications 1 à 8, **caractérisé en ce que** le dispositif capteur (16) pour mesurer l'espacement est un capteur à infrarouge.

11. Luminaire d'opération selon l'une des revendications 1 à 8, **caractérisé en ce que** le dispositif capteur (16) pour mesurer l'espacement est un capteur à laser.

12. Luminaire d'opération selon la revendication 11, **caractérisé en ce que** le capteur à laser irradie de la lumière dans la plage de longueurs d'onde visible.

13. Luminaire d'opération selon la revendication 11, **caractérisé en ce que** le capteur à laser irradie de la lumière dans la plage de longueurs d'onde non visible.

14. Luminaire d'opération selon la revendication 13, **caractérisé en ce que** le luminaire d'opération présente une source de rayon laser, qui irradie de la lumière dans la plage visible.

15. Luminaire d'opération selon la revendication 1, **caractérisé en ce que** plusieurs moyens d'éclairage (9) sont rassemblés en groupes, dont les faisceaux de rayons lumineux (11) présentent chacun un point d'intersection dans le plan du champ lumineux (13) afférent et les champs lumineux (13) présentent différents espacements par rapport au corps de luminaire (4).
